# FASCICULE DE BREVET EUROPEEN

(11) **EP 1 438 963 B1**
(45) Date de publication et mention de la délivrance du brevet: **04.07.2007**
(21) Numéro de dépôt: 04300008.2
(22) Date de dépôt: 06.01.2004
(51) Int. Cl.: A61K 33/00, A61P 25/00

(54) **Utilisation de N2O dans le traitement des détériorations cellulaires cérébrales post-ischémiques**
Verwendung von N2O zur Behandlung von postischemischen cerebralen zellulären Beschädigungen
Use of N2O for the treatment of postischemic cerebral cellular damages

(30) Priorité: 15.01.2003 FR 0350002
(43) Date de publication de la demande: 21.07.2004
(73) Titulaire: Air Liquide Santé (International), 75341 Paris Cedex 07 (FR)
(72) Inventeur: Lemaire, Marc, 75014 Paris (FR)
(74) Mandataire: Pittis, Olivier

(56) Documents cités:
- EP-A- 0 861 672
- WO-A-02/09731
- FR-A- 2 812 545
- DAVID H N ET AL: "REDUCTION OF ISCHEMIC BRAIN DAMAGE BY NITROUS OXIDE AND XENON" JOURNAL OF CEREBRAL BLOOD FLOW AND METABOLISM, RAVEN PRESS, LTD., NEW YORK, NY, US, vol. 23, no. 10, 1 octobre 2003 (2003-10-01), pages 1168-1173, XP008027594 ISSN: 0271-678X
- VERNA L. BAUGHMAN ET ALL.: "Neurologic Outcome in Rats Following Incomplete Cerebral Ischemia during Haloethane, Isoflurane or N2O" ANESTHESIOLOGY, vol. 69, 1988, pages 192-198, XP008030344
- WILLIAM E. HOFFMAN ET ALL.: "Interaction of Catecholamines and Nitrous Oxide Ventilation During Incomplete Brain Ischemia in Rats" ANESTHESIA & ANALGESIA, vol. 77, 1993, pages 908-912, XP008030343
- JEVTOVIC-TODOROVIC V. ET AL: 'NITROUS OXIDE (LAUGHING GAS) IS AN NMDA ANTAGONIST, NEUROPROTECTION AND NEUROTOXIN' NATURE MEDICINE vol. 4, no. 4, Avril 1998, NATURE AMERICA, NEW YORK, US, pages 460 - 463A, XP008049733
- YAMAKURA ET AL: 'Effects of Gaseous Aneesthetics Nitrous Oxide and Xenon on Ligan-gated Ion channels' ANESTHESIOLOGY vol. 93, no. 4, 2000, UNITED STATES, pages 1095 - 1101, XP008049734

## Description

L'invention porte sur l'utilisation de protoxyde d'azote (N₂O) et de xénon pour fabriquer tout ou partie d'un médicament destiné à traiter ou à prévenir une détérioration post-ischémique de cellules cérébrales, en particulier une détérioration consécutive à un accident vasculaire cérébral (AVC), notamment tout ou partie d'un médicament gazeux inhalable, chez l'homme ou l'animal.

Dans les ischémies cérébrales consécutives à un AVC et, de façon générale, dans les AVC, on note habituellement au plan neurochimique, une altération fonctionnelle de nombreux systèmes de neurotransmission, notamment une augmentation de la libération de glutamate dont l'excitotoxicité et la contribution à la mort neuronale sont connues, comme le rappelle Dirnagl et al., Trends Neurosci; 22 : 391, 1999.

Par ailleurs, au plan fonctionnel, on observe dans le cas d'ischémies globales chez le rat, une augmentation de l'activité locomotrice, notamment décrite par Wang et Corbett, Brain Res. 533: 78, 1990; Baldwin et al., Neurodegenration 2: 139, 1993, dont le développement est généralement attribué à une altération des fonctions cognitives de reconnaissance spatiales plutôt qu'à une altération des fonctions sensori-motrices.

De fait, le rôle thérapeutique potentiel des antagonistes des récepteurs glutamatergiques ionotropiques et métabotropiques a été soupçonné, notamment par Chazot, Curr Opin Invest Drugs 1: 370, 2000; Drian et al., Neurochem Int 38: 509, 2001.

Il apparaît aussi que les effets délétères des ischémies cérébrales avérées semblent impliquer des ischémies localisées qui auraient pour origine une excitotoxicité glutamatergique.

De fait, le thérapeutique potentiel des antagonistes des récepteurs glutamatergiques est souvent avancé dans le traitement de neuropathologies d'origine excitotoxique, en particulier les ischémies cérébrales, comme décrit par Dirnagl et al., Trends Neurosci 22: 391, 1999, et les troubles productifs, tel que décrit par *Benes, Brain Res. Review 31: 251, 2000.*

Cependant, la physiologie des récepteurs glutamatergiques est complexe et il semble que les antagonistes à haute affinité peuvent également présenter des propriétés neurotoxiques, selon Burns et al., Psychopharmacology 115: 516, 1994.

Ainsi, un intérêt thérapeutique potentiel des antagonistes à faible affinité, en particulier au NMDA, a été récemment proposé par Palmer et Widzowski, Amino acids 19: 151, 2000.

Par ailleurs, on connaît aussi le document WO-A-02/09731 portant sur l'utilisation de CO éventuellement additionné d'un autre gaz pour traiter les inflammations des voies aériennes supérieures ou des bronches. Ce document est donc ciblé sur le traitement des pathologies du type asthme, mucovicidose, pneumopathies ou analogues.

En outre, le document EP-A-861672 enseigne une méthode de traitement utilisable dans des situations d'urgence par administration de différents gaz. Toutefois, les dégradations cellulaires cérébrales post-ischémiques consécutives aux AVC ne sont pas concernées.

Enfin, le document FR-A-2812545 enseigne une association gaz et produit actif destinée à traiter ou prévenir la douleur. Le produit actif est un antalgique, un anti-inflammatoire, un antipyrétique ou analogue.

A ce jour, il n'existe donc pas de médicament efficace permettant de prévenir ou de traiter, au moins partiellement, les dégradations cellulaires cérébrales post-ischémiques consécutives aux accidents vasculaires cérébraux.

La présente invention s'inscrit dans ce contexte et vise à proposer toute ou partie d'un médicament utilisable pour prévenir, diminuer ou traiter toute détérioration post-ischémique de cellules cérébrales notamment consécutive à un accident vasculaire cérébral (AVC), chez l'homme ou l'animal.

L'invention concerne alors une utilisation de protoxyde d'azote (N₂O) et de xénon pour fabriquer tout ou partie d'un médicament destiné à traiter, à minimiser ou à prévenir une détérioration post-ischémique de cellules cérébrales.

Selon le cas, l'utilisation de l'invention peut comprendre l'une ou plusieurs des caractéristiques techniques suivantes :
- tout ou partie du médicament gazeux est sous forme inhalable.
- la détérioration post-ischémique cérébrale résulte ou est consécutive à un accident vasculaire cérébral (AVC).
- le protoxyde d'azote (N₂O) est sous forme gazeuse ou est compris dans un gaz ou un mélange gazeux.
- le médicament contient une proportion efficace de protoxyde d'azote (N₂O).
- le médicament contient, du xénon sous forme gazeuse ou compris dans un gaz ou un mélange gazeux.
- le médicament contient une proportion efficace de xénon.
- le médicament contient, en outre, au moins un autre composé gazeux choisi parmi l'oxygène, l'azote ou l'argon, de préférence de l'azote et de l'oxygène.
- le médicament contient une quantité inférieure à 60% en volume de xénon, de préférence inférieure ou égale à 50% en volume.
- le médicament contient une quantité allant jusqu'à environ 80% en volume de N₂O, de préférence jusqu'à 75% de N₂O.
- le médicament contient de 19 à 25% en volume d'oxygène et éventuellement de l'azote.

L'invention concerne alors aussi un médicament inhalable à action neuroprotectrice cérébrale contenant une quantité efficace de protoxyde d'azote (N₂O) et de xénon, en particulier destiné à traiter , à minimiser ou à prévenir une détérioration post-ischémique de cellules cérébrales.

Selon le cas, le médicament de l'invention peut comprendre l'une ou plusieurs des caractéristiques techniques suivantes :
- il contient une quantité allant jusqu'à 80% en volume de N₂O gazeux.
- il contient une quantité efficace de protoxyde d'azote (N₂O).
- il contient une quantité efficace de xénon.
- il contient une quantité inférieure à 60% en volume de xénon.
- il contient, en outre, de 19 à 25% en volume d'oxygène et éventuellement de l'azote.

L'idée à la base de la présente invention est de tirer partie des propriétés antagonistes des récepteurs NMDA du N₂O gazeux additionné de xénon, pour leur caractère neuroprotecteur dans la prévention ou le traitement de pathologies post-ischémiques consécutives aux accidents vasculaires cérébraux.

En effet, des travaux récents, réalisés in vitro, ont montré que le N₂O, ainsi que le xénon, peuvent potentiellement se comporter comme des antagonistes à faible affinité des récepteurs glutamatergiques au N-Méthyl-D-Aspartate NMDA (Franks et al., Nature 396: 324, 1998; Jevtovic-Todorovic et al., Nature Med. 4: 460, 199 ; Yamakura and Harris, Anesthesiology, 20008).

Partant de ces observations, des expérimentations ont été réalisées dans le cadre de la présente invention dans le but de déterminer les effets neuroprotecteurs du N₂O et du xénon sur la mort neuronale induite par ischémie cérébrale transitoire chez le rat.

Afin de démontrer l'effet bénéfique d'une administration de N₂O et de xénon sur les cellules cérébrales consécutivement à une ischémie cérébrale, des rats adultes Sprague-Dawley de 350 g ont été soumis au protocole expérimental suivant.

Au jour 1, des ischémies focalisées ont été induites chez chacun des rats par occlusion artérielle cérébrale moyenne (Middle Cerebral Artery Occlusion = MCAO), pendant une durée de 1 h 30 minutes.

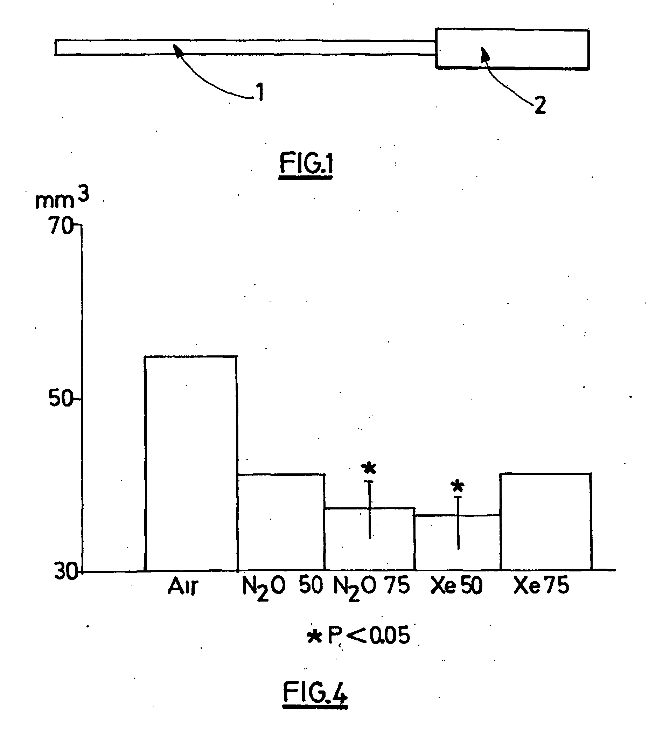

L'ischémie cérébrale focalisée transitoire par MCAO est obtenue de manière classique par introduction d'un fil 1 de nylon souple schématisé en Figure 1 (longueur 6.5 mm, diamètre 180 µm) dont une portion 2 de l'extrémité proximale est de diamètre supérieur à celui du fil (longueur 3 mm, diamètre 380 µm), à l'intérieur du système vasculaire du rat jusque dans la région de l'hémisphère ipsilatéral de manière à y provoquer une embolie, i.e. une ischémie.

Ensuite, on procède à une reperfusion des rats pendant 10 à 20 minutes, puis on leur fait inhaler plusieurs mélanges gazeux, à savoir :
- mélange n° 1 : air (témoin)
- mélange n° 2 : N₂O (75% vol) et le reste étant de l'oxygène (25%)
- mélange n° 3 : xénon (50 % vol) et le reste étant de l'oxygène (20 à 25%) et de l'azote (30 à 25%, respect.).
- mélange n° 4 : xénon (75% vol) et le reste étant de l'oxygène (25%)

Au jour 2, soit 24 heures après la reperfusion, les rats sont tués, les cerveaux sont récupérés, congelés, découpés en fines tranches de 40 µm d'épaisseur, puis colorés au violet cresyl, comme montré en Figure 5.

A partir des tranches obtenues après coloration, le volume de mort neuronal est calculé de manière classique en utilisant un logiciel classique adapté disponible dans le commerce.

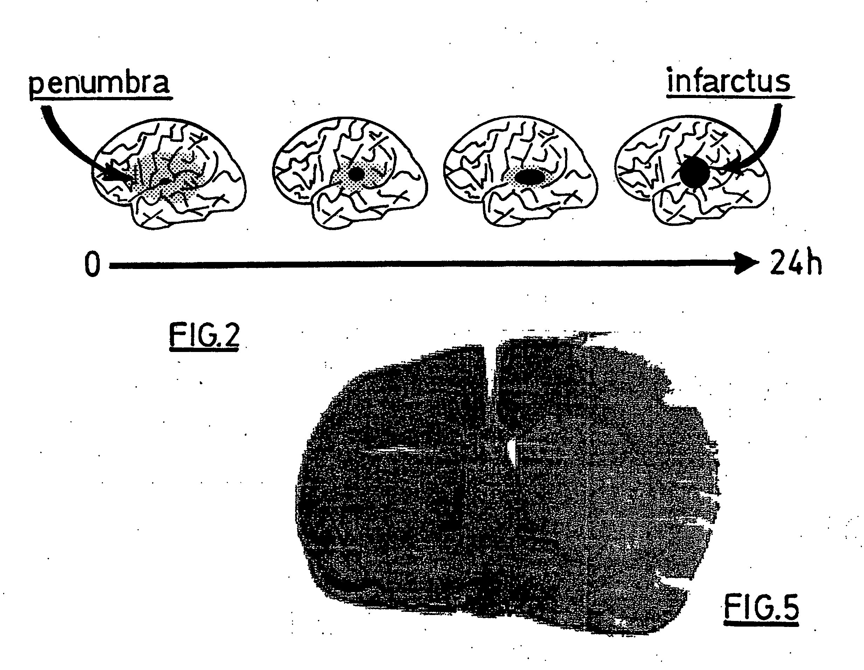

En effet, comme schématisé en figure 2, l'ischémie cérébrale engendre, en général, sous 24 heures, un infarctus dans la région ischémiée (penumbra) conduisant à une mort neuronale des cellules cérébrales présentes dans une partie importante de cette région.

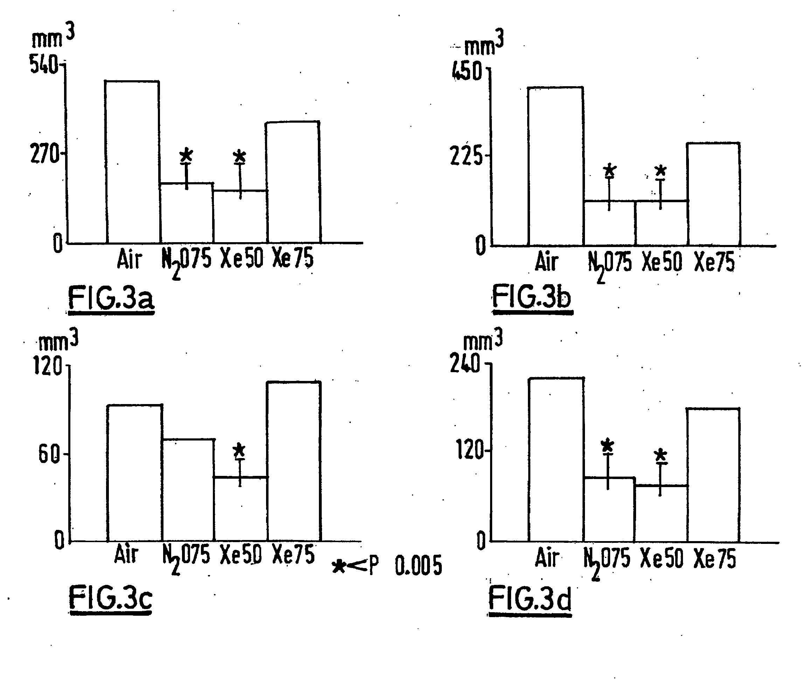

Les résultats obtenus lors de ces mesures ont été consignés sur les figures 3a à 3d qui permettent de visualiser l'effet neuroprotecteur post-ischémie cérébrale des mélanges n° 2 à 4 ci-dessus par comparaison au mélange n° 1 (air) servant de témoin.

Ainsi, la figure 3a montre clairement que l'inhalation par les rats de protoxyde d'azote (N₂O) ou de xénon (Xe) consécutivement à une ischémie permet de réduire notablement le volume total d'infarctus puisqu'une diminution de ce volume d'environ 50% peut être atteint en cas d'inhalation des mélanges n° 2 et n°3 en lieu et place de l'air (mélange n° 1 servant de témoin), et de 30% environ lors de l'inhalation du mélange n° 4.

A ce titre, on notera également que l'inhalation de 50% en volume de xénon (mélange n° 3) est plus efficace qu'une inhalation d'une dose plus élevée de xénon, à savoir 75% (mélange n° 4), ce qui laisse à penser que la dose la plus efficace semble être plus proche de 50% que de 75% pour ce qui concerne le xénon.

Les figures 3b à 3d confirment les résultats de la figure 3a puisqu'elles permettent de constater que l'inhalation de xénon ou de N₂O permet de diminuer, respectivement, le volume d'infarctus cortical (fig 3b), le volume d'infarctus striatal (fig. 3c) et le volume d'oedème (fig 3d) post-ischémiques par rapport à une inhalation d'air (témoin = mélange n° 1).

Partant de ce constat, des examens complémentaires ont été réalisés pour déterminer les effets neurotoxiques du xénon et du protoxyde d'azote (N₂O), à différentes teneurs, par rapport à l'air sur les récepteurs cérébraux de type NMDA.

Les résultats de ces examens sont consignés sur la figure 4 qui montre clairement que l'administration de xénon ou de protoxyde d'azote engendre un volume (en mn³) de récepteurs NMDA détériorés moins important que le témoin (air) et ce, avec le protoxyde d'azote dosé à 50% ou 75% en volume (reste = 25% de O₂) et le xénon dosé à 50% ou à 75% ( reste = mélange 25% de O₂ + 25% de N₂ ou, respect., 25% de O₂).

Toutefois, il apparaît ainsi un effet neurotoxique variable selon le dosage administré conduisant aux constats que le N₂O à 75% et le xénon à 50% en volume sont davantage neuroprotecteurs que le N₂O dosé à 50% et le xénon dosé à 75%.

Autrement dit, ces données confirment qu'une administration par inhalation de N₂O dosé à 75% en volume (ou moins) ou de xénon dosé à 50% en volume (ou moins) engendre une action neuroprotectrice vis-à-vis des ischémies cérébrales et autres maladies excitotoxiques analogues.

Le médicament inhalable selon l'invention est conditionné dans des récipients de gaz sous pression telles des bouteilles de gaz, et est distribué au patient via un système d'administration de gaz adapté muni d'un masque respiratoire, d'une sonde trachéale ou analogue.

Partant de là, dans le cadre de l'invention, on préférera l'utilisation de protoxyde d'azote (N₂O) gazeux, à celle du xénon, pour fabriquer un médicament destiné à traiter, à minimiser ou à prévenir les détériorations post-ischémiques de cellules cérébrales, tels les AVC.

## Revendications

1. - Utilisation de protoxyde d'azote (N₂O) et de xénon pour fabriquer tout ou partie d'un médicament gazeux destiné à traiter, à minimiser ou à prévenir une détérioration post-ischémique de cellules cérébrales.

2. - Utilisation selon la revendication 1, **caractérisée en ce que** tout ou partie du médicament gazeux est sous forme inhalable.

3. - Utilisation selon l'une des revendications 1 ou 2, **caractérisée en ce que** la détérioration post-ischémique cérébrale résulte ou est consécutive à un accident vasculaire cérébral (AVC).

4. - Utilisation selon l'une des revendications 1 à 3, **caractérisée en ce que** le médicament contient du xénon sous forme gazeuse ou du xénon compris dans un gaz ou un mélange gazeux.

5. - Utilisation selon l'une des revendications 1 à 4, **caractérisée en ce que** le protoxyde d'azote (N₂O) est sous forme gazeuse ou est compris dans un gaz ou un mélange gazeux.

6. - Utilisation selon l'une des revendications 1 à 5, **caractérisée en ce que** le médicament contient une proportion efficace de protoxyde d'azote (N₂O) et de xénon.

7. - Utilisation selon l'une des revendications 1 à 6, **caractérisée en ce que** le médicament contient, en outre, au moins un autre composé gazeux choisi parmi l'oxygène, l'azote ou l'argon, de préférence de l'azote et de l'oxygène.

8. - Utilisation selon l'une des revendications 1 à 7, **caractérisée en ce que** le médicament contient une quantité inférieure à 60% en volume de xénon, de préférence inférieure ou égale à 50% en volume.

9. - Utilisation selon l'une des revendications 1 à 8, **caractérisée en ce que** le médicament contient une quantité allant jusqu'à 80% en volume de N₂O, de préférence jusqu'à 75% de N₂O.

10. - Utilisation selon l'une des revendications 1 à 9, **caractérisée en ce que** le médicament contient de 19 à 25% en volume d'oxygène et éventuellement de l'azote.

11. - Médicament inhalable à action neuroprotectrice cérébrale contenant une quantité efficace de protoxyde d'azote (N₂O) et de xénon.

12. - Médicament selon la revendication 11, **caractérisé en ce qu**'il contient une quantité allant jusqu'à 80% en volume de N₂O.

13. - Médicament selon la revendication 11, **caractérisé en ce qu**'il contient du xénon en une quantité inférieure à 60% en volume.

14. - Médicament selon l'une des revendications 11 à 13, **caractérisé en ce qu**'il contient, en outre, de 19 à 25% en volume d'oxygène et éventuellement de l'azote.

15. - Récipient de gaz sous pression contenant un médicament selon l'une des revendications 11 à 14, en particulier une bouteille de gaz.

## Claims

1. Use of nitrous oxide (N₂O) and of xenon, for producing all or part of a gaseous medicinal product intended to treat, minimize or prevent post-ischaemic brain cell deterioration.

2. Use according to Claim 1, **characterized in that** all or part of the gaseous medicinal product is in inhalable form.

3. Use according to either of Claims 1 and 2, **characterized in that** the post-ischaemic brain deterioration results in or is subsequent to a stroke.

4. Use according to one of Claims 1 to 3, **characterized in that** the medicinal product contains xenon in gaseous form or xenon included in a gas or mixture of gases.

5. Use according to one of Claims 1 to 4, **characterized in that** the nitrous oxide (N₂O) is in gaseous form or is included in a gas or in a mixture of gases.

6. Use according to one of Claims 1 to 5, **characterized in that** the medicinal product contains an effective proportion of nitrous oxide (N₂O) and of xenon.

7. Use according to one of Claims 1 to 6, **characterized in that** the medicinal product also contains at least one other gaseous compound chosen from oxygen, nitrogen or argon, preferably nitrogen and oxygen.

8. Use according to one of Claims 1 to 7, **characterized in that** the medicinal product contains an amount which is less than 60% by volume of xenon, preferably less than or equal to 50% by volume.

9. Use according to one of Claims 1 to 8, **characterized in that** the medicinal product contains an amount ranging up to 80% by volume of N₂O, preferably up to 75% of N₂O.

10. Use according to one of Claims 1 to 9, **characterized in that** the medicinal product contains from 19 to 25% by volume of oxygen and, optionally, of nitrogen.

11. Inhalable medicinal product with neuroprotective action in the brain, containing an effective amount of nitrous oxide (N₂O) and of xenon.

12. Medicinal product according to Claim 11, **characterized in that** it contains an amount ranging up to 80% by volume of N₂O.

13. Medicinal product according to Claim 11, **characterized in that** it contains xenon in an amount which is less than 60% by volume.

14. Medicinal product according to one of Claims 11 to 13, **characterized in that** it also contains from 19 to 25% by volume of oxygen and, optionally, of nitrogen.

15. Pressurized gas container containing a medicinal product according to one of Claims 11 to 14, in particular a gas bottle.

## Patentansprüche

1. Verwendung von Distickstoffmonoxid (N₂O) und Xenon zur Herstellung eines gasförmigen Arzneimittels insgesamt oder eines Teils desselben, wobei dieses Arzneimittel dazu bestimmt ist, postischämische Schäden an Hirnzellen zu behandeln, diesen vorzubeugen oder diese so gering wie möglich zu halten.

2. Verwendung nach Anspruch 1, **dadurch gekennzeichnet, dass** das gasförmige Arzneimittel insgesamt oder ein Teil desselben in inhalierbarer Form vorliegt.

3. Verwendung nach einem der Ansprüche 1 oder 2, **dadurch gekennzeichnet, dass** die postischämischen Hirnschäden durch einen Schlaganfall (CVA) entstehen oder in Folge eines solchen auftreten.

4. Verwendung nach einem der Ansprüche 1 bis 3, **dadurch gekennzeichnet, dass** das Arzneimittel gasförmiges Xenon oder ein Gas oder eine Gasmischung, das/die Xenon umfasst, enthält.

5. Verwendung nach einem der Ansprüche 1 bis 4, **dadurch gekennzeichnet, dass** das Distickstoffmonoxid (N₂O) als Gas vorliegt oder Teil eines Gases oder einer Gasmischung ist.

6. Verwendung nach einem der Ansprüche 1 bis 5, **dadurch gekennzeichnet, dass** das Arzneimittel einen wirksamen Anteil an Distickstoffmonoxid (N₂O) oder Xenon enthält.

7. Verwendung nach einem der Ansprüche 1 bis 6, **dadurch gekennzeichnet, dass** das Arzneimittel darüber hinaus eine weitere gasförmige Verbindung enthält, die aus Sauerstoff, Stickstoff oder Argon, vorzugsweise jedoch aus Stickstoff und Sauerstoff, gewählt ist.

8. Verwendung nach einem der Ansprüche 1 bis 7, **dadurch gekennzeichnet, dass** das Arzneimittel Xenon in einer Menge von weniger als 60 Volumen%, vorzugsweise von nicht mehr als 50 Volumen%, enthält.

9. Verwendung nach einem der Ansprüche 1 bis 8, **dadurch gekennzeichnet, dass** das Arzneimittel N₂O in einer Menge von bis zu 80 Volumen%, vorzugsweise von bis zu 75 Volumen%, enthält.

10. Verwendung nach einem der Ansprüche 1 bis 9, **dadurch gekennzeichnet, dass** das Arzneimittel 19 bis 25 Volumen% an Sauerstoff sowie möglicherweise Stickstoff enthält.

11. Inhalierbares Arzneimittel mit neuroprotektiver Wirkung auf das Gehirn, wobei das Arzneimittel eine wirksame Menge an Distickstoffmonoxid (N₂O) und an Xenon enthält.

12. Arzneimittel nach Anspruch 11, **dadurch gekennzeichnet, dass** es N₂O in einer Menge von bis zu 80 Volumen% enthält.

13. Arzneimittel nach Anspruch 11, **dadurch gekennzeichnet, dass** es Xenon in einer Menge von weniger als 60 Volumen% enthält.

14. Arzneimittel nach einem der Ansprüche 11 bis 13, **dadurch gekennzeichnet, dass** es darüber hinaus 19 bis 25 Volumen% an Sauerstoff sowie möglicherweise Stickstoff enthält.

15. Druckbehälter für Gase, welcher ein Arzneimittel nach einem der Ansprüche 11 bis 14 enthält, wobei es sich bei dem Druckbehälter insbesondere um eine Gasflasche handelt.
